(19) Europäisches Patentamt
European Patent Office
Office européen des brevets

(11) **EP 3 410 939 B1**

(12) **FASCICULE DE BREVET EUROPEEN**

(45) Date de publication et mention de la délivrance du brevet:
**04.03.2020   Bulletin 2020/10**

(51) Int Cl.:
*A61B 6/00* (2006.01)          *G09B 23/28* (2006.01)
*G09B 23/30* (2006.01)

(21) Numéro de dépôt: **17701693.8**

(22) Date de dépôt: **26.01.2017**

(86) Numéro de dépôt international:
**PCT/EP2017/051605**

(87) Numéro de publication internationale:
**WO 2017/133957 (10.08.2017 Gazette 2017/32)**

(54) **FANTOME THYROIDIEN, PROCEDE DE FABRICATION CORRESPONDANT, FANTOME GLOBAL COMPRENANT UN TEL FANTOME THYROIDIEN ET FAMILLES DE FANTOMES CORRESPONDANTES**

SCHILDDRÜSENPHANTOM, ENTSPRECHENDES HERSTELLUNGSVERFAHREN, ALLGEMEINES PHANTOM MIT SOLCH EINEM SCHILDDRÜSENPHANTOM UND ENTSPRECHENDE PHANTOMFAMILIEN

THYROID PHANTOM, CORRESPONDING PRODUCTION METHOD, OVERALL PHANTOM COMPRISING SUCH A THYROID PHANTOM AND CORRESPONDING PHANTOM FAMILIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.02.2016   FR 1650855**

(43) Date de publication de la demande:
**12.12.2018   Bulletin 2018/50**

(73) Titulaire: **Institut de Radioprotection et de Sûreté Nucléaire
92260 Fontenay aux Roses (FR)**

(72) Inventeurs:
• **BROGGIO, David
  92140 Clamart (FR)**
• **BEAUMONT, Tiffany
  94800 Villejuif (FR)**

(74) Mandataire: **Decorchemont, Audrey Véronique Christèle et al
CABINET BOETTCHER
16, rue Médéric
75017 Paris (FR)**

(56) Documents cités:
EP-A1- 2 977 008          DE-C1- 19 628 997
JP-A- 2014 215 222        US-A- 5 227 627

• Anonymous: "Thyroid Phantom", , 7 août 2014 (2014-08-07), XP055304837, Extrait de l'Internet: URL:http://www.jzimaging.com/Biodex_thyroid_phantom.htm [extrait le 2016-09-22]
• Anonymous: "Med Equipment Platform Thyroid phantom", , 1 janvier 2014 (2014-01-01), XP055304851, Extrait de l'Internet: URL:http://images.google.de/imgres?imgurl= http://www.medwow.com/med/nuclear-medicine -phantom/nuclear-associates/74-340-thyroid /x74-340-thyroid.mth21500_200_200.jpg.page speed.ic.sRZh5qSuUm.jpg&imgrefurl=http://w ww.medwow.com/med/nuclear-medicine-phant om /nuclear-associates/74-340-thyroid/21500.m odel-spec& [extrait le 2016-09-22]

## Description

L'invention concerne un fantôme thyroïdien. L'invention concerne aussi un procédé de fabrication d'un tel fantôme thyroïdien. L'invention concerne également un fantôme global comprenant un tel fantôme thyroïdien. L'invention concerne également une famille comprenant plusieurs tels fantômes thyroïdien. L'invention concerne également une famille comprenant plusieurs tels fantômes globaux.

## ARRIERE PLAN TECHNOLOGIQUE DE L'INVENTION

L'anthroporadiométrie de la thyroïde consiste à mesurer des rayonnements de type X ou $\gamma$ émis par la thyroïde lors de la désintégration de radionucléides contenus dans la thyroïde. La mesure expérimentale ainsi acquise est comparée à une mesure de référence, encore appelée mesure d'étalonnage, ce qui permet de déduire les différents radionucléides présents dans la thyroïde et leurs activités.

La mesure de référence est usuellement obtenue en effectuant une étude anthroporadiométrique sur un ou des fantômes numériques ou physiques. Ces fantômes sont des objets numériques ou physiques représentant plus ou moins schématiquement la thyroïde et sont généralement remplis d'une solution jouant le rôle d'une source d'émission de rayonnements, source qui s'avère de nature et d'activité connue.

Ce type de fantôme thyroïdien est d'ailleurs utilisé dans d'autres domaines d'imagerie et de dosimétrie médicale que l'anthroporadiométrie.

Document JP2014215222 divulgue un fantôme comprenant un élément correspondant à la thyroïde, ce fantôme comportant au moins deux parties définissant entre elles une cavité et des moyens de fixation des deux parties entre elles pour fermer de manière étanche cette cavité et des moyens de remplissage de la cavité d'une solution et des moyens de bouchage temporaire hermétique.

## OBJET DE L'INVENTION

Un but de l'invention est de proposer un fantôme thyroïdien physique relativement simple de fabrication et d'utilisation qui s'avère en outre relativement réaliste du point de vue anatomique. Un but de l'invention est également de proposer un procédé de fabrication d'un tel fantôme thyroïdien. Un but de l'invention est également de proposer un fantôme global comprenant un tel fantôme thyroïdien. Un but de l'invention est également de proposer une famille comprenant plusieurs tels fantômes thyroïdien. Un but de l'invention est également de proposer une famille comprenant plusieurs tels fantômes globaux.

## BREVE DESCRIPTION DE L'INVENTION

En vue de la réalisation de ce but, on propose un fantôme thyroïdien, le fantôme comprenant un corps comportant au moins deux parties définissant entre elles une empreinte d'une thyroïde et des moyens de fixation des deux parties entre elles pour fermer de manière étanche l'empreinte, le fantôme comprenant en outre des moyens de remplissage de l'empreinte d'une solution, lesdits moyens de remplissage comprenant au moins un canal s'étendant depuis l'extérieur du corps jusqu'à dans l'empreinte et des moyens de bouchage temporaire hermétique dudit canal.

De la sorte, le fantôme thyroïdien peut être fabriqué relativement facilement par création indépendante de ces deux parties de corps et fixation de ces deux parties l'une à l'autre. En particulier, les deux parties du corps du fantôme thyroïdien peuvent être fabriquées séparément par impression en trois dimensions ce qui permet une fabrication relativement aisée et reproductible du fantôme.

De plus, une fois monté, le fantôme peut tout aussi bien être rempli de solution que laissé vide. Il s'avère ainsi possible de fournir à un client directement le fantôme ou le fantôme avec une solution particulière insérée dans le fantôme. On peut ainsi envisager de remplir le fantôme d'une solution particulière, par exemple une solution radioactive, fermer le fantôme de façon hermétique au niveau du canal de remplissage, avant de livrer le tout à un client qui n'a pas la possibilité ou l'habilitation de se fournir directement pour cette solution.

Ceci facilite l'utilisation du fantôme par le client.

De plus, le fantôme s'avère relativement réaliste d'un point de vue anatomique grâce à l'empreinte de la thyroïde.

L'invention concerne également un procédé de fabrication d'un tel fantôme comportant les étapes successives suivantes : modélisation numérique en trois dimensions du corps du fantôme thyroïdien, fabrication de façon séparée de chaque partie du corps par impression en trois dimensions à l'aide d'une imprimante 3D, nettoyage de chaque partie du corps ainsi créée d'un matériau perdu utilisé par l'imprimante 3D pour former l'empreinte, assemblage des deux parties du corps entre elles.

L'invention concerne également une famille de fantôme thyroïdien comportant au moins deux fantômes thyroïdiens tels que décrits précédemment, chaque fantôme thyroïdien étant adapté à simuler au moins la thyroïde d'une catégorie d'êtres humains particulière distincte.

L'invention concerne également un fantôme global comprenant un fantôme thyroïdien tel que décrit précédemment, le fantôme global comprenant un fantôme de cou recevant le fantôme thyroïdien, un fantôme de vertèbres cervicales reçu dans le fantôme de cou et un fantôme de moelle épinière reçu dans le fantôme de vertèbres cervicales.

L'invention concerne également une famille de fantôme global comportant au moins deux fantômes globaux tels que décrits précédemment, chaque fantôme global étant adapté à simuler au moins la thyroïde d'une catégorie d'êtres humains particulière distincte.

BREVE DESCRIPTION DES DESSINS

L'invention sera mieux comprise à la lumière de la description qui suit d'un mode de réalisation particulier non limitatif de l'invention.

Il sera fait référence aux figures ci-jointes parmi lesquelles :

- la figure 1 est une vue en perspective du fantôme thyroïdien selon un mode de réalisation particulier de l'invention, les éléments internes du fantôme étant visibles en pointillés,
- la figure 2 est une vue en coupe en perspective du fantôme illustré à la figure 1, la vue étant une vue éclatée dudit fantôme,
- la figure 3 est une vue de face de la coupe représentée à la figure 2,
- la figure 4 est un schéma représentant les différentes étapes du procédé de fabrication du fantôme thyroïdien tel qu'illustré à la figure 1,
- la figure 5 est une vue en perspective d'une série de fantômes thyroïdiens, famille comprenant le fantôme thyroïdien tel qu'illustré à la figure 1,
- la figure 6 est une vue éclatée en perspective d'un fantôme global comprenant le fantôme thyroïdien illustré à la figure 1,
- la figure 7 est une vue en perspective d'une série de fantômes globaux comprenant chacun respectivement l'un des fantômes thyroïdiens de la famille illustrée à la figure 5.

DESCRIPTION DETAILLEE DE L'INVENTION

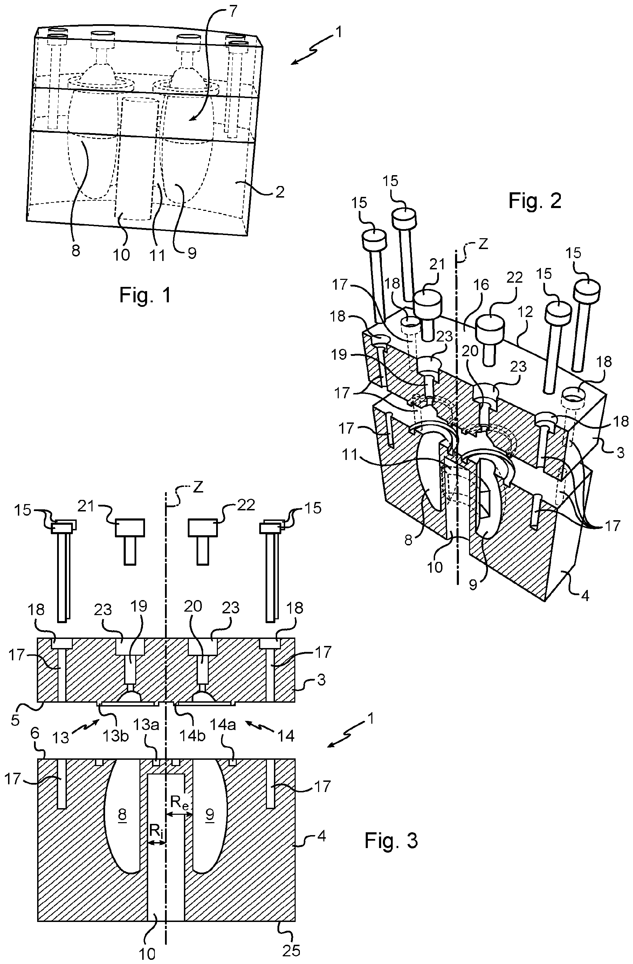

En référence aux figures 1 à 3, le fantôme thyroïdien selon l'invention, généralement désigné en 1, est ici destiné à être utilisé pour un étalonnage de mesures anthroporadiométriques. Cette application n'est bien sûr pas limitative et on pourra utiliser ledit fantôme dans le cadre d'autres applications comme pour l'étalonnage dans d'autres formes d'imageries et de dosimétries notamment, bien que non exclusivement dans le domaine médical, comme dans la médecine nucléaire ou la radioprotection.

Le fantôme thyroïdien 1 comprend un corps 2 qui est ici composé d'une première partie 3 et d'une deuxième partie 4. La face inférieure 5 de la première partie 3 repose sur la face supérieure 6 de la deuxième partie 4, lesdites faces étant de mêmes dimensions. Le corps 2 est ainsi conformé de sorte que lesdites faces (et donc la frontière entre les deux parties) s'étendent dans un plan de normale un axe Z. L'axe Z est ici sensiblement vertical, c'est-à-dire que lesdites faces s'étendant sensiblement horizontalement. L'axe Z symbolise ainsi la direction tête-pieds d'un organisme humain.

Le corps 2 est de préférence dans un matériau apte à être utilisé par une imprimante en trois dimensions.

Le corps 2 est ici en matériau polymère.

De préférence, le corps 2 est dans un matériau qui est en outre transparent. Ceci permet de surveiller les différents éléments internes au corps 2 que nous verrons par la suite.

De préférence, le corps 2 est dans un matériau ayant des propriétés de transmission des rayons X ou gamma (y) proches de celle des tissus humains.

Le corps 2 est par exemple à base de polyméthacrylate de méthyle ou de polycarbonate.

Les deux parties 3, 4 sont conformées de sorte à définir entre elles une empreinte 7 fermée et représentative d'une thyroïde d'un organisme humain. On rappelle que la thyroïde d'un organisme humain a une forme générale de papillon et est ainsi composée de deux lobes symétriques situés de part et d'autre de la trachée, les deux lobes étant liés par une bande de tissu appelée isthme. L'empreinte 7 est ici conformée de sorte à comporter deux lobes 8, 9 séparés l'un de l'autre par un cylindre 10 d'axe Z du corps 2 simulant une trachée humaine (le cylindre ne faisant donc pas partie de l'empreinte 7).

De préférence, ledit cylindre 10 est creusé intérieurement de sorte à former un tube s'étendant selon l'axe Z et débouchant uniquement au niveau de la face inférieure 25 de la deuxième partie 4 (l'autre extrémité du cylindre 10 étant donc non débouchant au niveau de la face supérieure 6 de la deuxième partie 4). Ainsi formé, le cylindre 10 ne débouche donc pas dans l'empreinte 7. Le cylindre 10 présente un rayon externe $R_e$ et un rayon interne $R_i$. Chaque lobe 8, 9 présente ainsi ici une forme générale d'elliposoïde d'axe de révolution parallèle à l'axe Z (les deux lobes 8, 9 sont ainsi verticaux) délimité au niveau de sa surface en regard de l'autre lobe par la surface cylindrique d'axe Z. Les deux lobes 8, 9 sont donc symétriques relativement à l'axe Z. Chaque lobe 8, 9 est par exemple défini à partir des équations générales suivantes :

$$(\frac{x - x_0}{a})^2 + (\frac{y - y_0}{b})^2 + (\frac{z - z_0}{c})^2 \leq 1$$

$$x^2 + (y - y_0)^2 \geq R_e^{\;2}$$

$R_e$ étant le rayon externe de la trachée et $x_0$, $y_0$, $z_0$, a, b et c des paramètres définissant le lobe concerné.

L'empreinte 7 est en outre conformée de sorte à relier les extrémités inférieures des deux lobes 8, 9 par un isthme 11. L'isthme 11 est ici conformé en une portion d'anneau dont une extrémité débouche dans l'un des lobes 8 et dont l'autre extrémité débouche dans l'autre des lobes 9. L'isthme 11 n'est toutefois pas débouchant dans le cylindre 10.

La portion d'anneau est ainsi ici agencée de sorte à avoir un centre de révolution agencé sur l'axe Z et à présenter un rayon interne égal à $R_e$. La face interne de la portion d'anneau assure donc une continuité de l'empreinte 7 entre la face incurvée du premier lobe 8 et la face incurvée du deuxième lobe 9.

Cette portion d'anneau est par exemple définie à partir des équations suivantes :

$$R_e^2 \leq x^2 + (y - y_0)^2 \leq R_{isth}^2$$

$$z - h_{isth} \leq z \leq z_0$$

$$y \leq y_0$$

$R_{isth}$ étant le rayon externe de la portion d'anneau ($R_e$ étant le rayon interne de la portion d'anneau égal au rayon externe du cylindre 10) et $h_{isth}$ la hauteur de la portion d'anneau.

L'empreinte 7 ainsi décrite comporte les deux lobes 8, 9 et l'isthme 11 et est fermée.

Les équations précitées décrivant l'empreinte sont issues du modèle d'Ulanovsky et d'Eckerman (décrit en particulier dans l'article « Absorbed fractions for electron and photon émissions in the developing thyroid : fetus to five years old » publié dans la revue Radiation Protection Dosimetry Vol. 79, Nos 1-4, pp. 419-424 en 1998) .

De préférence, ces modèles d'Ulanovsky et d'Eckerman sont adaptés pour suivre les nouvelles recommandations de la Commission Internationale de Protection Radiologique (plus connue sous les acronymes CIPR ou ICRP en anglais) étant entendu que les modèles d'Ulanovsky et d'Eckerman sont basés sur d'anciennes recommandations de la CIPR. Cette adaptation peut par exemple se faire en appliquant un facteur d'échelle aux modèles d'Ulanovsky et d'Eckerman pour suivre les nouvelles recommandations.

De préférence également, ces modèles d'Ulanovsky et d'Eckerman sont adaptés pour prendre en compte la nature d'une solution qui serait éventuellement agencée dans l'empreinte 7 notamment une solution acide et/ou radioactive. On veillera ainsi à ce que la distance séparent l'empreinte 7 de l'extérieur soit suffisamment importante pour que les parois correspondantes puissent résister à l'attaque de la solution (notamment au niveau de la paroi formant le cylindre 10) .Les deux parties 3, 4 du corps 2 sont ici agencées de sorte que leur frontière s'étende à travers les extrémités supérieures des deux lobes 8, 9. Les lobes 8, 9 sont donc majoritairement ménagés dans la deuxième partie 4 et minoritairement dans la première partie 3 et l'isthme 11 est entièrement ménagé dans la deuxième partie 4 du corps 2.

Selon un mode de réalisation préféré, le corps 2 est conformé de sorte à présenter globalement une forme de parallélépipède rectangle dont une face latérale principale 12 (par opposition aux faces supérieure et inférieure du corps 2 de normale l'axe Z) est arrondie pour simuler la courbure d'un cou d'un organisme humain.

Ceci permet au fantôme thyroïdien 1 d'être mieux représentatif d'un organisme humain ce qui permet d'obtenir de meilleures mesures de références à l'aide dudit fantôme.

De préférence, le corps 2 ici aussi conformé de sorte à prendre en considération la distance thyroïde-cou d'un organisme humain c'est-à-dire la distance séparant l'isthme de la thyroïde de la surface du cou (dans le cas du corps 2 ladite distance est simulée par la distance séparant l'empreinte 7 de la face latérale principale 12).

De préférence encore, le corps 2 est également conformé de sorte à prendre en considération l'atténuation des rayons émis par une thyroïde humaine dans les tissus adipeux entourant ladite thyroïde.

Ainsi, la position de l'empreinte 7 relativement à la face latérale arrondie 12 du corps 2 est configurée de sorte à prendre en compte à la fois une distance thyroïde-cou d'un organisme humain, le matériau formant le fantôme thyroïdien 1 et les caractéristiques physiques des tissus adipeux d'un organisme humain, comme nous le verrons par la suite.

Ceci permet au fantôme thyroïdien 1 d'être mieux représentatif d'un organisme humain ce qui permet d'obtenir de meilleures mesures de références à l'aide dudit fantôme.

Le fantôme thyroïdien 1 comporte des moyens de fixation des deux parties 3, 4 du corps entre elles pour fermer de manière hermétique l'empreinte 7.

Ainsi, le fantôme thyroïdien 1 est conformé de sorte à pouvoir être rempli d'une solution.

De préférence, les moyens de fixation comportent un premier système d'emboîtement 13 et un deuxième système d'emboîtement 14 comprenant chacun une portion femelle 13a, 14a et une portion mâle 13b, 14b, l'une des portions étant portée par la première partie 3 au niveau de la frontière entre les deux parties 3, 4 et l'autre des portions étant portée par la deuxième partie 4 en regard de l'autre portion au niveau de la frontière entre les deux parties 3, 4 pour assurer l'emboîtement des deux portions entre elles.

Ceci permet de bien imbriquer les deux parties du corps 2. On améliore ainsi l'étanchéité du fantôme thyroïdien 1 en particulier au niveau d'une zone critique qu'est la frontière de l'empreinte 7.

De façon particulière, les portions femelles 13a, 14a sont des orifices en forme de boucle (selon l'axe Z) ménagées dans l'une des parties et les portions mâles 13b, 14b sont des boucles correspondantes en relief s'étendant à partir de l'autre des parties. Les portions sont agencées de sorte que le premier système d'emboîtement 13 entoure l'extrémité supérieure du premier lobe 8 et que le deuxième système d'emboîtement 14 entoure l'extrémité supérieure du deuxième lobe 9. Ainsi pour chaque système d'emboîtement 13, 14, la portion mâle et la portion femelle entoure chacune le lobe correspondant sur respectivement l'une ou l'autre de la face supérieure 6 de la deuxième partie 4 et la face inférieure 5 de la première partie 3.

Les boucles sont par exemple en forme d'anneau circulaire ou en forme d'anneau elliptique .

De façon particulière, les moyens de fixation comportent en outre des vis 15 assurant la solidarisation étanche des deux parties 3, 4 entre elles.

Les moyens de fixation comportent par exemple quatre vis 15 qui sont agencées aux quatre coins de la face supérieure 16 de la première partie 3 et dont les tiges s'étendent, depuis la première partie 3 jusque dans la deuxième partie 4, selon une direction respective parallèle à la direction Z. Les vis 15 sont agencées de sorte que les surfaces supérieures des têtes de vis affleurent la face supérieure 16 de la première partie 3. Le corps 2 comporte donc quatre orifices 17 pour recevoir les tiges des vis 15 (et donc ménagés dans la première partie 3 et dans la deuxième partie 4) et quatre logements 18 en extrémité supérieure desdits orifices 17 (et donc ménagés dans la première partie 3) pour recevoir les têtes de vis correspondantes.

De préférence, les moyens de fixation comportent en outre de la colle (non visible ici) agencée entre la tête de chaque vis 15 et le fond du logement 18 recevant ladite tête de vis 15.

Ceci permet d'améliorer la fixation des deux parties 3, 4 du corps 2.

De préférence, ladite colle est dans un matériau résistant aux acides.

La colle est ici à base de polyméthacrylate de méthyle, de polycarbonate ou d'un polyépoxyde (soit un mélange d'une résine époxyde et d'un durcisseur). La colle est par exemple de la colle Araldite (marque déposée).

De façon préférée, les moyens de fixation comportent en outre de la colle (non visible ici) agencée à l'intérieur des systèmes d'emboitement 13, 14.

Ceci permet d'améliorer la fixation des deux parties 3, 4 du corps 2.

De préférence, ladite colle est dans un matériau résistant aux acides.

La colle est ici à base de polyméthacrylate de méthyle, de polycarbonate ou d'un polyépoxyde (soit un mélange d'une résine époxyde et d'un durcisseur). La colle est par exemple de la colle Araldite (marque déposée).

Le fantôme thyroïdien 1 comprend en outre des moyens de remplissage de l'empreinte 7 (les deux lobes 8, 9 et l'isthme 11) d'une solution depuis l'extérieur du fantôme thyroïdien 1.

Lesdits moyens de remplissage comprennent un premier canal 19 s'étendant depuis la face supérieure 16 de la première partie 3 jusque dans l'extrémité supérieure du premier lobe 8 et un deuxième canal 20 s'étendant depuis la face supérieure 16 de la première partie 3 jusque dans l'extrémité supérieure du deuxième lobe 9.

Les moyens de remplissage comportent également des moyens de bouchage temporaire hermétique du premier canal 19 et du deuxième canal 20.

A cet effet, les moyens de bouchage comportent une première vis 21 pour boucher le premier canal 19 et une deuxième vis 22 pour boucher le deuxième canal 20. Les moyens de remplissage sont ainsi conformés de sorte que les surfaces supérieures des têtes de vis affleurent la face supérieure 16 de la première partie 3 du corps 2. Les deux canaux 19, 20 comportent donc chacun un logement 23 à l'extrémité supérieure du canal concerné pour recevoir la tête de vis correspondante.

Les vis 21, 22 des moyens de remplissage sont de préférence dans un matériau résistant aux acides. De préférence, les vis 21, 22 des moyens de remplissage sont en outre dans un matériau ne diffusant ou n'absorbant pas ou peu les rayons X ou gamma $\gamma$.

De la sorte, le fantôme thyroïdien 1 est conformé de sorte à pouvoir être rempli d'une solution radioactive.

Ceci permet au fantôme thyroïdien 1 de fournir des mesures d'étalonnages encore plus précises lors d'une étude anthroporadiométrique.

De préférence, les vis 15 des moyens de blocage sont également dans un matériau résistant aux acides. De préférence également, les vis 15 des moyens de blocage ne diffusent ou n'absorbent pas ou peu les rayons X ou gamma.

Toutes les vis des moyens de blocage et des moyens de remplissage 15, 21, 22 sont par exemple en nylon.

Les moyens de bouchage comportent également un premier joint (non visible ici) agencé dans le premier canal 19 entre la tête de vis 21 et le fond du logement 23 et compressé par ladite tête de vis 21 contre le fond du logement 23 et un deuxième joint (non visible ici) agencé dans le deuxième canal 20 entre la tête de vis 22 et le fond du logement 23 et compressé par ladite tête de vis 22.

De préférence, lesdits joints sont dans un matériau de dureté inférieure à celle formant le corps 2. De préférence également, lesdits joints sont dans un matériau résistant à l'acide.

Ceci permet d'assurer une étanchéité durable.

Les joints sont par exemple à base de caoutchouc. Par exemple, les joints sont à base de caoutchouc synthétique de la famille des élastomères fluorés. Les joints sont ici en Viton (marque déposée).

Selon un mode de réalisation préféré, le fantôme thyroïdien 1 contient une solution (non visible ici) emplissant l'empreinte 7 du fantôme thyroïdien 1.

La solution est préférentiellement une solution radioactive.

De préférence, la solution comprend au moins un radionucléide simulant au moins l'une des raies d'émission de l'Iode 131 ($^{131}$I) et présentant une durée de vie plus longue que l'Iode 131.

En effet, l'Iode 131 est un radionucléide rejeté en forte concentration dans l'atmosphère en cas d'incident nucléair se fixant facilement sur la thyroïde humaine. En outre le spectre d'émission de l'Iode 131 comporte une raie d'énergie relativement élevée à 365 kiloélectron-volts (82% d'émission) qui n'est pas ou peu atténuée par les tissus d'un organisme humain notamment les tissus adipeux entourant une thyroïde. Dès lors la détection de l'Iode 131 est relativement aisée par anthroporadiométrie. Il est donc intéressant que le fantôme thyroïdien 1 puisse être adapté à fournir une mesure de référence en lien avec ce radionucléide. Toutefois, l'Iode 131 a une durée de vie de sensiblement 8.0228 jours ce qui n'est pas suffisant pour que l'Iode 131 puisse être utilisé directement dans la solution remplissant le fantôme thyroïdien.

De façon particulière, la solution comporte comme radionucléide le Baryum 133 ($^{133}$Ba).

Un tel radionucléide a une durée de vie de sensiblement 10 ans et demi ce qui lui permet de pouvoir être employé dans le fantôme thyroïdien. En outre, le Baryum 133 a un spectre d'émission présentant une raie d'énergie également élevée de 356 kiloélectron-volts (62% d'émission) de sorte à simuler correctement le comportement de l'Iode 131.

La concentration du Baryum 133 dans la solution est par exemple comprise entre 100 et 130 becquerel par gramme pour simuler une thyroïde d'un enfant et entre 35 et 40 becquerel par gramme pour simuler une thyroïde d'un adulte.

De préférence, la solution comporte en outre au moins un composant acide afin de placer le Baryum 133 dans un milieu acide.

Ceci permet d'assurer une stabilité et une homogénéité de la solution due à la présence du radionucléide Baryum 133.

Le composant acide est par exemple de l'acide chlorhydrique. La concentration de l'acide chlorhydrique dans la solution est comprise par exemple entre 2 et 16% et de préférence entre 2 et 5%.

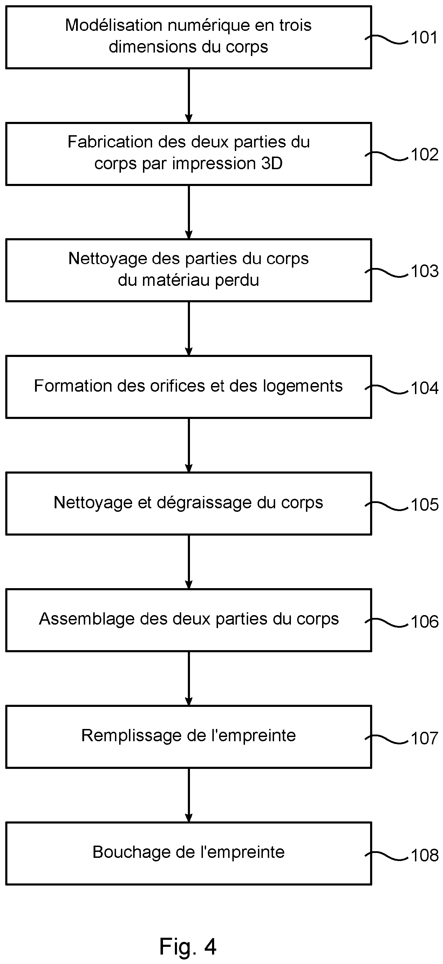

En référence à la figure 4, le procédé de fabrication du fantôme thyroïdien 1 va être à présent décrit.

La première étape 101 consiste à créer une modélisation numérique en trois dimensions du corps 2 du fantôme thyroïdien 1, en s'aidant des équations citées précédemment, pour modéliser l'empreinte 7, à savoir :

$$\left(\frac{x - x_0}{a}\right)^2 + \left(\frac{y - y_0}{b}\right)^2 + \left(\frac{z - z_0}{c}\right)^2 \leq 1$$

$$x^2 + (y - y_0)^2 \geq R_e^{\,2}$$

$$R_e^2 \leq x^2 + (y - y_0)^2 \leq R_{isth}^2$$

$$z - h_{isth} \leq z \leq z_0$$

$$y \leq y_0$$

La position de l'empreinte 7 dans le corps 2 est également modélisée en tenant donc compte d'une distance thyroïde-cou d'un organisme humain, du matériau formant le fantôme thyroïdien 1 et des caractéristiques physiques du tissu adipeux d'un organisme humain, à l'aide de la relation suivante :

$$x = x_{tissu} * (\mu_{tissu}/\mu)$$

avec x la distance thyroïde-cou du fantôme thyroïdien 1, $x_{tissu}$ la distance thyroïde-cou d'un organisme humain, $\mu$ le coefficient d'atténuation linéique du matériau dans lequel on souhaite former le corps du fantôme thyroïdien 1 et $\mu_{tissu}$ le coefficient d'atténuation linéique du tissu adipeux d'un organisme humain entourant une thyroïde humaine.

On adapte en outre les dimensions du corps 2, de l'empreinte 7 ainsi que la position relative de l'empreinte 7 dans le corps 2 en fonction du type de catégorie de personnes que l'on souhaite viser lors des mesures effectuées par le fantôme thyroïdien 1 ainsi réalisé. Par exemple, si on souhaite créer un fantôme thyroïdien 1 d'un homme adulte, les différentes dimensions et position relative de l'empreinte 7 dans le corps 2 seront différentes que si l'on souhaite créer un fantôme thyroïdien 1 d'un enfant.

Les recommandations de la CIPR permettent notamment d'adapter les équations précitées au type de patient visé au niveau des dimensions du cylindre 10 du corps 2, de l'empreinte 7 ainsi que la position relative de l'empreinte 7 dans le corps 2.

En particulier, lesdites recommandations et le type de raie d'énergie que l'on souhaite étudier avec le fantôme thyroïdien permettent d'estimer $x_{tissu}$ et $\mu_{tissu}$. On retient par exemple un coefficient d'atténuation linéique du tissu adipeux $\mu_{tissu}$ de 0.1056 centimètres$^{-1}$ pour une raie d'énergie élevée de 356 kiloélectron-volts (correspondante à celle du Baryum 133 utilisé dans le cas présent). On retient en outre par exemple, pour un adulte, une distance thyroïde-cou $x_{tissu}$ de 0.5 centimètres.

La deuxième étape 102 consiste à fabriquer de façon séparée chaque partie 3, 4 du corps 2 par impression en trois dimensions à l'aide d'une imprimante 3D. De façon connue en soi, l'imprimante 3D va utiliser un matériau perdu, tel qu'un gel ou une résine, pour assurer la formation de l'empreinte 7 à l'intérieur des parties 3, 4 du corps 2.

La troisième étape 103 consiste à nettoyer chaque partie 3, 4 du corps 2 ainsi créée du matériau perdu recouvrant ou remplissant lesdites parties. Cette étape 103 est par exemple mise en œuvre à l'aide d'un jet d'eau sous haute pression. On note que la construction du corps 2 en deux parties 3, 4 permet de facilement et rapidement nettoyer ledit corps 2 du matériau perdu.

La quatrième étape 104 consiste à former les orifices (par forage par exemple) et les logements destinés à recevoir les quatre vis 15 des moyens de fixation et les deux vis 21, 22 des moyens de remplissage.

Une cinquième étape 105 consiste à nettoyer et à dégraisser le corps.

La sixième étape 106 consiste à assembler les deux parties 3, 4 du corps 2 entre elles. On pose la colle au niveau de chaque logement 18 et dans les systèmes d'emboitement 13 et 14, on imbrique ensuite les deux parties 3, 4 du corps 2 entre elles au niveau des systèmes d'emboîtement 13, 14 puis on agence les vis 15 des moyens de fixation à l'intérieur des orifices 17. On note que l'imbrication des deux corps 3, 4 au niveau des systèmes d'emboîtement 13, 14 facilite la pose ultérieure des vis 15.

La septième étape 107 consiste ici à remplir l'empreinte 7 de la solution. Par exemple la solution est contenue dans une seringue qui est insérée dans le corps 2 successivement au niveau de chaque canal de remplissage 19, 20 jusqu'à atteindre l'empreinte 7. Le contenu de la seringue est alors vidé dans l'empreinte 7.

La huitième étape 108 consiste enfin à boucher les canaux de remplissage 19, 20 en posant d'abord les joints puis on visant les vis 21, 22 dans les deux canaux 19, 20 pour fermer hermétiquement l'empreinte 7.

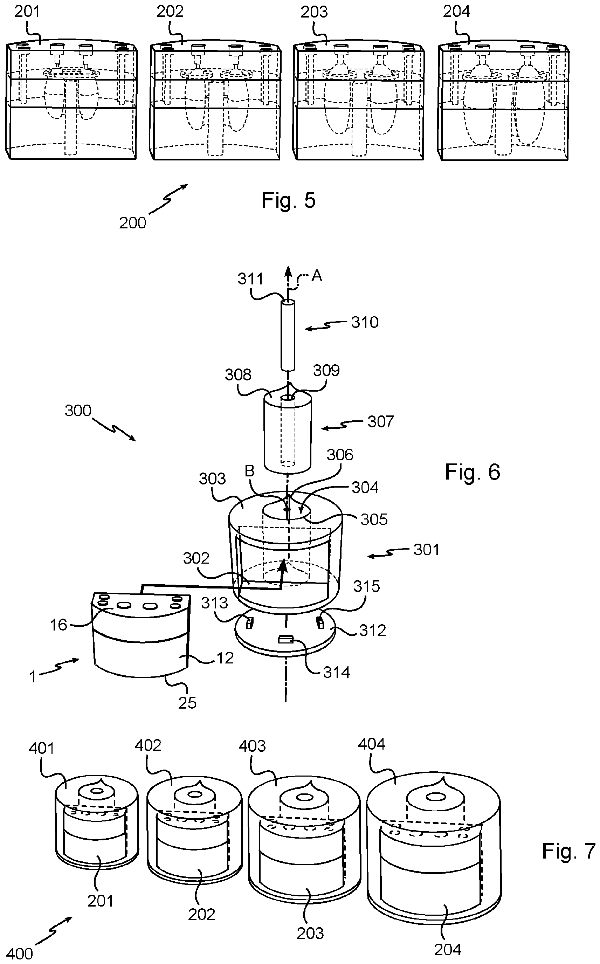

De préférence, comme visible à la figure 5, le procédé selon l'invention est mis en œuvre de sorte à fabriquer une famille 200 de fantôme thyroïdien tel que précédemment décrit, chaque fantôme étant destiné à servir d'étalon pour des mesures de références pour une catégorie d'âge de patient particulier. Ici, une famille 200 de quatre fantômes est fabriquée : un premier fantôme 201 associé à un patient de cinq ans, un deuxième fantôme 202 associé à un patient de 10 ans, un troisième fantôme 203 associé à un patient de 15 ans et un quatrième fantôme 204 associé à un patient adulte.

Les dimensions de chaque fantôme (notamment dimensions de l'empreinte, du corps et de la distance isthme-face arrondie 12 du corps symbolisant la distance cou-thyroïde fixée par les propriétés de transmission du matériau du fantôme 1) sont ainsi adaptées de sorte à simuler la thyroïde d'un patient de l'âge concerné par le fantôme.

On peut ainsi réaliser de multiples mesures de références. De façon avantageuse, chaque fantôme thyroïdien s'avère ainsi plus représentatif d'un organisme humain d'une classe particulière ce qui permet d'obtenir de meilleures mesures de références à l'aide dudit fantôme.

En référence à la figure 6, de façon préférée, le fantôme thyroïdien 1 est inséré dans un fantôme global 300.

Le fantôme global 300 comporte un fantôme de cou 301, destiné à simuler le cou d'un organisme humain comprenant la thyroïde, qui est ici conformé en un cylindre d'axe de révolution A parallèle à l'axe Z.

Le cylindre est par exemple défini par les équations suivantes :

$$ x^2 + y^2 \leq R_H^2 $$

$$ C_T \ \leq z \leq C_T \ + C_{HO} $$

avec $R_H$ le rayon du cylindre et $C_{HO}$ la hauteur du cylindre, $C_T$ correspondant à une distance pied-tronc d'un patient associé audit fantôme de cou.

Les équations du cou précitées et les valeurs de $C_T$ sont décrites plus en détails par exemple dans l'article « Description of the mathematical phantoms » disponible à la page https://crpk.ornl.gov/resources/Mird.pdf et publié par le Oak Ridge National Laboratory en 2005.

Le fantôme de cou 301 comprend un premier logement 302 dans lequel est reçu le fantôme thyroïdien 1. Ce premier logement 302 est formé dans le cylindre de sorte que la face latérale arrondie 12 du fantôme thyroïdien 1 forme elle-même une portion de la face latérale du cylindre, la face latérale du fantôme thyroïdien 1 opposée à cette face arrondie 12 reposant alors contre l'intérieur du cylindre. Le premier logement 302 est en outre conformé de sorte que la face supérieure 16 de la première partie 3 du fantôme thyroïdien 1 repose contre l'intérieur du cylindre et de sorte que la face inférieure 25 de la deuxième partie 4 du fantôme thyroïdien 1 repose contre l'intérieur du cylindre.

De façon particulière, le fantôme global 300 comporte un socle 312 sur lequel repose le fantôme de cou 301.

Le socle 112 est ici conformé en un cylindre d'axe de révolution A de même rayon $R_H$ que le rayon du cylindre du fantôme de cou 301. Le socle 112 forme ainsi un prolongement du fantôme de cou 301.

De préférence, le fantôme de cou 301 comporte des moyens de fixation sur le socle 312.

Typiquement, les moyens de fixation comportent un premier système d'emboîtement 313, un deuxième système d'emboitement 314 et un troisième système d'emboitement 315. Chaque système d'emboitement comporte une portion femelle et une portion mâle, l'une des portions étant portée par la face inférieure du fantôme de cou 301 et l'autre des portions étant portée par la face supérieure du socle 312 pour assurer l'emboîtement du fantôme de cou 301 et du socle 312 entre eux.

Ceci permet de bien imbriquer le fantôme 301 et le socle 312.

De préférence, les moyens de fixation comportent en outre de la colle agencée entre le fantôme de cou 301 et le socle 312.

La colle est par exemple à base de polyépoxyde, de polyméthacrylate de méthyle ou de polycarbonate.

Le socle 112 est de préférence dans un matériau n'influant pas sur des mesures effectuées à partir du fantôme global 300. Le socle 112 est ainsi ici en matière plastique. Le socle 112 est par exemple en polyméthacrylate de méthyle, en polycarbonate ou encore en polyépoxyde.

De préférence, le fantôme de cou 301 comporte un deuxième logement 304 central qui s'étend selon l'axe A et qui débouche d'une part au niveau de la face supérieure 303 du fantôme de cou 301 et d'autre part au niveau de la face inférieure du fantôme de cou 301 et donc du socle 312. Ce deuxième logement 304 a une section (selon un plan de coupe de normale l'axe A) en forme de goutte dont la partie centrale 305 est conformée en une ellipse de centre B agencé sur l'axe A et dont la partie en pointe 306 s'étend selon une direction radiale, en direction opposée au premier logement 302.

Le fantôme global 300 comporte un fantôme des vertèbres cervicales 307, fantôme qui est de forme complémentaire au deuxième logement 304 et qui s'étend donc selon l'axe A dans le fantôme de cou 301. Le fantôme des vertèbres cervicales 307 est ainsi agencé de sorte que sa face inférieure repose sur le socle 312 et que sa face supérieure 308 soit au même niveau que la face supérieure 303 du fantôme de cou 301. On note que la partie en pointe de ce fantôme des vertèbres cervicales 307 permet de simuler l'apophyse épineuse.

De préférence, le fantôme des vertèbres cervicales 307 comporte un logement central 309 qui s'étend selon l'axe A et qui débouche au niveau de la face supérieure 308 du fantôme des vertèbres cervicales 307 et au niveau de la face inférieure du fantôme des vertèbres cervicales 307. Ce logement 309 a une section (selon un plan de coupe de normale l'axe A) circulaire de centre agencé sur l'axe A.

Le fantôme global 300 comporte en outre un fantôme de moelle épinière 310, fantôme qui est de forme complémentaire au logement 309 et qui s'étend donc selon l'axe A dans le fantôme des vertèbres cervicales 307. Le fantôme de moelle épinière 310 est ainsi agencé de sorte que sa face inférieure repose sur le socle 312 et que sa face supérieure 311 soit au même niveau que la face supérieure 308 du fantôme des vertèbres cervicales 307.

De préférence, chaque fantôme du fantôme global 300 est dans un matériau adapté à simuler au mieux les caractéristiques physiques (en particulier ici les propriétés de transmission des rayons X ou gamma) de l'organe qu'il est censé représenter. Pour le fantôme de cou 301 et le fantôme thyroïdien 1, on vise un matériau simulant au mieux les tissus humains comme déjà expliqué. Pour le fantôme des vertèbres cervicales 307, on vise un matériau simulant au mieux les os et pour le fantôme de la moelle épinière 310, on vise un matériau simulant au mieux la moelle épinière.

Par exemple pour le fantôme des vertèbres cervicales 307, une vertèbre humaine est formée d'un os spongieux entouré d'un os dur (cortical). Si l'on détermine la densité moyenne de ces deux os, en considérant la proportion d'os spongieux et d'os cortical donnée par les recommandations de la CIPR, on obtient pour un homme adulte une densité moyenne de 1.427 grammes par centimetres$^{-3}$ et donc un coefficient d'atténuation linéique de 0.152 de centimètre$^{-1}$ pour une raie d'énergie élevée de 356 kiloélectron-volts (correspondante à celle du Baryum 133 utilisé dans le cas présent). On cherche ainsi un matériau présentant un coefficient d'atténuation linéique proche de cette valeur.

De la sorte, le fantôme global 300 comporte le fantôme de cou 301, le socle 312, le fantôme thyroïdien 1, le fantôme des vertèbres cervicales 307 et le fantôme de moelle épinière 310.

Une étude anthroporadiométrique ou de médecine nucléaire, peut ainsi être faite sur le fantôme thyroïdien 1 seul, ou bien sur le fantôme global 300 incluant obligatoirement le fantôme thyroïdien et tout ou partie du reste du fantôme global 300.

On peut ainsi réaliser de multiples mesures dans des conditions de références différentes. De façon avantageuse, le fantôme global 300 est plus représentatif d'un organisme humain ce qui permet d'obtenir de meilleures mesures de références à l'aide dudit fantôme notamment en ce qui concerne le fantôme thyroïdien 1.

De façon préférée, comme visible à la figure 7, lorsqu'une famille 200 de fantômes thyroïdiens est créée comme illustrée à la figure 5, chacun de ces fantômes thyroïdiens est inséré respectivement dans un fantôme global.

On a donc ainsi une famille de fantôme global 400 tel que précédemment décrit, chaque fantôme étant destiné à servir d'étalon pour des mesures de références pour une catégorie d'âge de patient particulier. Ici, une famille 400 de quatre fantômes est donc fabriquée en liaison avec la famille 200 de fantômes thyroïdiens décrite en référence avec la figure 5 : un premier fantôme 401 associé à un patient de cinq ans, un deuxième fantôme 402 associé à un patient de 10 ans, un troisième fantôme 403 associé à un patient de 15 ans et un quatrième fantôme 404 associé à un patient adulte.

Les dimensions de chaque fantôme global (et donc de chaque fantôme thyroïdien, fantôme de cou, fantôme des vertèbres cervicales et fantôme de moelle épinière ainsi que le socle) sont ainsi adaptées de sorte à simuler un cou, une thyroïde, une moelle épinière et des vertèbres cervicales d'un patient de l'âge concerné par le fantôme.

On peut ainsi réaliser de multiples mesures de références. De façon avantageuse, chaque fantôme global s'avère ainsi plus représentatif d'un organisme humain d'une classe particulière ce qui permet d'obtenir de meilleures mesures de références à l'aide dudit fantôme.

L'invention n'est pas limitée à ce qui vient d'être décrit, mais bien au contraire englobe toute variante entrant dans le cadre défini par les revendications.

En particulier, bien qu'ici les différents fantômes (thyroïdiens et globaux) soient adaptés à un âge particulier les fantômes pourront en complément ou en remplacement également être adaptés à un sexe particulier féminin ou masculin ou encore en complément ou en remplacement également être adaptés à une pathologie particulière notamment, bien que non exclusivement, liée à la thyroïde (hyperthyroïde ...).

Les différents matériaux cités pourront bien entendu être différents de ce qui a été décrit. Bien entendu, lorsque l'on souhaite remplir l'empreinte d'une solution, on veillera à choisir les matériaux du corps, des moyens de fixation et des moyens de remplissage de sorte à ce qu'ils soient compatibles avec cette solution ou du moins suffisamment résistants à cette solution pour que le fantôme demeure étanche et hermétique et ce pour une durée de vie d'au moins plusieurs annnées et de préférence d'au moins un an. La solution pourra tout aussi bien être liquide ou semi-liquide (par exemple être une résine ou encore un gel).

Bien qu'ici la solution comporte du Baryum 133, la solution pourra en remplacement ou en complément comprendre tout autre radionucléide notamment, bien que non exclusivement, un radionucléide ayant un intérêt dans les domaines d'applications médicales (anthroporadiamétrie, médecine nucléaire, radioprotection ...). La solution pourra ainsi par exemple comprendre un couple Baryum 133 et Cesium 137. Par ailleurs, la solution pourra comprendre un radionucléide simulant au moins l'une des raies d'émission d'un autre radionucélide que l'Iode 131 notamment, bien que non exclusivement, un radionucléide ayant un intérêt dans les domaines d'applications médicales (anthroporadiamétrie, médecine nucléaire, radioprotection ...) tel que l'Iode 123.

Bien qu'ici, mis à part en termes de dimensions, les fantômes thyroïdiens soient tous identiques au sein d'une même famille, les fantômes pourront être légèrement différents au sein d'une même famille. On pourra ainsi préférer une boucle englobant les deux lobes pour les fantômes thyroïdiens de plus petites dimensions (par exemple pour les enfants de 5 ans) et des boucles entourant chacune l'un des lobes pour les fantômes thyroïdiens de plus grandes dimensions (par exemple pour les enfants de plus de 5 ans et pour les adultes). On pourra aussi adapter la forme des canaux de remplissage au volume de l'empreinte à remplir de solution.

De la même façon, bien qu'ici mis à part en termes de dimensions, les fantômes globaux soient tous identiques au sein d'une même famille, les fantômes pourront être légèrement différents au sein d'une même famille.

Bien qu'ici à une famille de fantômes thyroïdiens soit associée une famille de fantômes globaux, à une famille de fantômes thyroïdiens pourra être associé un unique fantôme global, ledit fantôme global étant conformé de sorte à recevoir de façon individuel chacun des fantômes thyroïdiens de la famille de fantômes thyroïdiens.

Les différents fantômes pourront être différents de ce qui a été décrit. Par exemple, bien qu'ici le fantôme thyroïdien comporte un cylindre en creux, le fantôme thyroïdien pourra ne pas comporter un tel cylindre, la trachée étant alors par exemple simplement représentée par l'espace séparant les lobes de l'empreinte. Le fantôme global pourra ne pas comporter de socle, les différents fantômes étant par exemple tous reçus dans des logements du fantômes de cou présentant des fonds fermés.

**Revendications**

1. Fantôme thyroïdien (1 ; 201, 202, 203, 204), le fantôme comprenant un corps (2) comportant au moins deux parties (3,4) définissant entre elles une empreinte (7) d'une thyroïde et des moyens de fixation des deux parties entre elles pour fermer de manière étanche l'empreinte, le fantôme comprenant en outre des moyens de remplissage de l'empreinte d'une solution, lesdits moyens de remplissage comprenant au moins un canal (19, 20) s'étendant depuis l'extérieur du corps jusqu'à dans l'empreinte et des moyens de bouchage temporaire hermétique dudit canal.

2. Fantôme selon la revendication 1, dans lequel le corps (2) comporte un cylindre creux (10) s'étendant dans le corps entre deux lobes de l'empreinte (7) sans toutefois traverser l'empreinte.

3. Fantôme selon la revendication 1, dans lequel le corps (2) est dans un matériau apte à être utilisé par une imprimante en trois dimensions.

4. Fantôme selon l'une des revendications précédentes, dans lequel le corps (2) est conformé de sorte à présenter globalement une forme de parallélépipède rectangle, une face latérale principale (12) étant arrondie.

5. Fantôme selon l'une des revendications précédentes, dans lequel l'empreinte (7) est agencée dans le corps (2) de sorte à prendre en compte une distance thyroïde-cou d'un organisme humain.

6. Fantôme selon l'une des revendications précédentes, dans lequel l'empreinte (7) est agencée dans le corps (2) de sorte à prendre en compte une atténuation de rayons émis par une thyroïde dans des tissus d'un organisme humain.

7. Fantôme selon l'une des revendications précédentes, dans lequel les moyens de fixation comportent au moins un système d'emboîtement (13, 14) comprenant une portion femelle et une portion mâle, l'une des portions étant portée par la première partie (3) et l'autre des portions étant portée par la deuxième partie (4) en regard de l'autre portion pour assurer l'emboîtement des deux portions entre elles.

**8.** Fantôme selon la revendication 7, dans lequel le système d'emboîtement (13, 14) entoure au moins l'un des lobes (8, 9) de l'empreinte.

**9.** Fantôme selon l'une des revendications précédentes, dans lequel les moyens de fixation comportent au moins une vis (15) assurant la solidarisation étanche des deux parties de corps (3, 4) entre elles.

**10.** Fantôme selon la revendication 9, dans lequel les moyens de fixation comportent en outre de la colle agencée entre la vis et un orifice du corps (2) recevant la vis.

**11.** Fantôme selon l'une des revendications précédentes, dans lequel les moyens de remplissage comprennent un premier canal (19) s'étendant depuis la face supérieure (16) de la première partie (3) jusque dans une extrémité supérieure d'un premier lobe (8) de l'empreinte et un deuxième canal (20) s'étendant depuis la face supérieure de la première partie (4) jusque dans une extrémité supérieure d'un deuxième lobe (9) de l'empreinte.

**12.** Fantôme selon la revendication 11, dans lequel les moyens de bouchage comportent deux vis (21, 22) bouchant respectivement l'un des canaux et deux joints, chaque joint étant agencé dans le canal (19, 20) considéré entre la vis (21, 22) associée et ledit canal.

**13.** Fantôme selon l'une des revendications précédentes, dans lequel le fantôme comporte une solution emplissant l'empreinte du fantôme thyroïdien.

**14.** Fantôme selon la revendication 13, dans lequel la solution est une solution radioactive.

**15.** Fantôme selon la revendication 14, dans lequel la solution comprend au moins un radionucléide simulant au moins l'une des raies d'émission de l'Iode 131 et présentant une durée de vie plus longue que l'Iode 131.

**16.** Procédé de fabrication d'un fantôme selon l'une des revendications précédentes, comportant les étapes successives suivantes :

- modélisation numérique en trois dimensions du corps du fantôme thyroïdien (101),
- fabrication de façon séparée de chaque partie du corps par impression en trois dimensions à l'aide d'une imprimante 3D (102),
- nettoyage de chaque partie du corps ainsi créée d'un matériau perdu utilisé par l'imprimante 3D pour former l'empreinte (103),
- formations des différents orifices et logements (105) destinés à recevoir les moyens de bouchage et les moyens de fixation,
- assemblage des deux parties du corps entre elles (106).

**17.** Famille de fantôme thyroïdien (200) comportant au moins deux fantômes thyroïdiens selon l'une des revendications 1 à 15, chaque fantôme thyroïdien étant adapté à simuler au moins la thyroïde d'une catégorie d'êtres humains particulière distincte.

**18.** Fantôme global (300) comprenant un fantôme thyroïdien selon l'une des revendications à 1 à 15, le fantôme global comprenant un fantôme de cou (301) recevant le fantôme thyroïdien, un fantôme de vertèbres cervicales (307) reçu dans le fantôme de cou et un fantôme de moelle épinière (310) reçu dans le fantôme de vertèbres cervicales.

**19.** Fantôme global (300) selon la revendication 18, comprenant en outre un socle (312) sur lequel est agencé le fantôme de cou.

**20.** Famille de fantôme global (400) comportant au moins deux fantômes globaux selon la revendication 18 ou la revendication 19, chaque fantôme global étant adapté à simuler au moins la thyroïde d'une catégorie d'êtres humains particulière distincte.

**Patentansprüche**

**1.** Schilddrüsenphantom (1; 201, 202, 203, 204), wobei das Phantom einen Körper (2) umfasst, der mindestens zwei Teile (3, 4) hat, die zwischen sich eine Hohlform (7) einer Schilddrüse definieren, und Befestigungsmittel zum Befestigen der beiden Teile miteinander, um die Hohlform auf dichte Weise zu verschließen, wobei das Phantom ferner Füllmittel zum Befüllen der Hohlform mit einer Lösung umfasst, wobei die genannten Füllmittel mindestens einen Kanal (19, 20) umfassen, der sich von der Außenseite des Körpers bis in die Hohlform erstreckt, sowie Verschlussmittel zum temporären hermetischen Verschließen des Kanals.

**2.** Phantom nach Anspruch 1, bei dem der Körper (2) einen hohlen Zylinder (10) umfasst, der sich in dem Körper zwischen zwei Lappen der Hohlform (7) erstreckt, ohne jedoch durch die Hohlform hindurchzugehen.

**3.** Phantom nach Anspruch 1, bei dem der Körper (2) aus einem Material ist, das geeignet ist, von einem dreidimensionalen Drucker verwendet zu werden.

**4.** Phantom nach einem der vorhergehenden Ansprüche, bei dem der Körper (2) derart geformt ist, dass er insgesamt eine Form eines rechteckigen Parallelepipeds aufweist, wobei eine Hauptseitenfläche (12) abgerundet ist.

5. Phantom nach einem der vorhergehenden Ansprüche, bei dem die Hohlform (7) in dem Körper (2) derart ausgebildet ist, dass ein Abstand Schilddrüse-Hals eines menschlichen Organismus berücksichtigt wird.

6. Phantom nach einem der vorhergehenden Ansprüche, bei dem die Hohlform (7) in dem Körper (2) derart angeordnet ist, dass eine Dämpfung von Strahlen, die von einer Schilddrüse in Gewebe eines menschlichen Organismus ausgesendet werden, berücksichtigt wird.

7. Phantom nach einem der vorhergehenden Ansprüche, bei dem die Befestigungsmittel mindestens ein Stecksystem (13, 14) umfassen, das einen weiblichen Abschnitt und einen männlichen Abschnitt umfasst, wobei einer der Abschnitte von dem ersten Teil (3) und der andere der Abschnitte von dem zweiten Teil (4) gegenüber dem anderen Abschnitt getragen wird, um das Ineinanderstecken der beiden Abschnitte zu gewährleisten.

8. Phantom nach Anspruch 7, bei dem das Stecksystem (13, 14) mindestens einen der Lappen (8, 9) der Hohlform umgibt.

9. Phantom nach einem der vorhergehenden Ansprüche, bei dem die Befestigungsmittel mindestens eine Schraube (15) umfassen, die die dichte Befestigung der beiden Körperteile (3, 4) miteinander gewährleistet.

10. Phantom nach Anspruch 9, bei dem die Befestigungsmittel ferner Klebstoff umfassen, der zwischen der Schraube und einer Öffnung des Körpers (2), die die Schraube aufnimmt, angeordnet ist.

11. Phantom nach einem der vorhergehenden Ansprüche, bei dem die Füllmittel einen ersten Kanal (19) umfassen, der sich von der oberen Fläche (16) des ersten Teils (3) bis in ein oberes Ende eines ersten Lappens (8) der Hohlform erstreckt, sowie einen zweiten Kanal (20), der sich von der oberen Fläche des ersten Teils (4) bis in ein oberes Ende eines zweiten Lappens (9) der Hohlform erstreckt.

12. Phantom nach Anspruch 11, bei dem die Verschlussmittel zwei Schrauben (21, 22) umfassen, die jeweils einen der Kanäle verschließen, sowie zwei Dichtungen, wobei jede Dichtung in dem betreffenden Kanal (19, 20) zwischen der dazugehörigen Schraube (21, 22) und dem genannten Kanal angeordnet ist.

13. Phantom nach einem der vorhergehenden Ansprüche, bei dem das Phantom eine Lösung umfasst, die die Hohlform des Schilddrüsenphantoms füllt.

14. Phantom nach Anspruch 13, bei dem die Lösung eine radioaktive Lösung ist.

15. Phantom nach Anspruch 14, bei dem die Lösung mindestens ein Radionuklid umfasst, das mindestens eine der Emissionslinien von Jod 131 simuliert und eine längere Lebensdauer als Jod 131 aufweist.

16. Verfahren zur Herstellung eines Phantoms nach einem der vorhergehenden Ansprüche, umfassend die folgenden aufeinanderfolgenden Schritte:

 - digitale Modellierung in drei Dimensionen des Körpers des Schilddrüsenphantoms (101),
 - getrennte Herstellung jedes Teils des Körpers durch dreidimensionalen Druck mit Hilfe eines 3D-Druckers (102),
 - Säuberung jedes so erzeugten Teils des Körpers von einem verlorenen Material, das von dem 3D-Drucker zum Bilden der Hohlform verwendet wird (103),
 - Ausbildung verschiedener Öffnungen und Aufnahmen (105), die dazu bestimmt sind, die Verschlussmittel und die Befestigungsmittel aufzunehmen,
 - Zusammenfügung der beiden Teile des Körpers miteinander (106).

17. Schilddrüsenphantomfamilie (200), umfassend mindestens zwei Schilddrüsenphantome nach einem der Ansprüche 1 bis 15, wobei jedes Schilddrüsenphantom geeignet ist, mindestens die Schilddrüse einer besonderen unterscheidbaren Kategorie von Menschen zu simulieren.

18. Gesamtphantom (300), umfassend ein Schilddrüsenphantom nach einem der Ansprüche 1 bis 15, wobei das Gesamtphantom ein Halsphantom (301) umfasst, das das Schilddrüsenphantom aufnimmt, ein Halswirbelphantom (307), das in dem Halsphantom aufgenommen ist, und ein Rückenmarksphantom (310), das in dem Halswirbelphantom aufgenommen ist.

19. Gesamtphantom (300) nach Anspruch 18, ferner umfassend einen Sockel (312), auf dem das Halsphantom angeordnet ist.

20. Gesamtphantomfamilie (400), umfassend mindestens zwei Gesamtphantome nach Anspruch 18 oder Anspruch 19, wobei jedes Gesamtphantom geeignet ist, mindestens die Schilddrüse einer besonderen unterscheidbaren Kategorie von Menschen zu simulieren.

## Claims

1. A thyroid phantom (1; 201, 202, 203, 204), the phantom comprising a body (2) comprising at least two parts (3, 4) between them defining a thyroid cavity (7) and means for fixing the two parts together to close the cavity in a sealed manner, the phantom further comprising means for filling the cavity with a solution, said filling means comprising at least one canal (19, 20) extending from outside the body into the cavity and means for temporarily hermetically plugging said canal.

2. The phantom as claimed in claim 1, in which the body (2) comprises a hollow cylinder (10) extending in the body between two lobes of the cavity (7) without, however, passing through the cavity.

3. The phantom as claimed in claim 1, in which the body (2) is made from a material capable of being used by a three-dimensional printer.

4. The phantom as claimed in one of the preceding claims, in which the body (2) is configured in such a way as to exhibit the overall shape of a rectangular parallelepiped, one main lateral face (12) being rounded.

5. The phantom as claimed in one of the preceding claims, in which the cavity (7) is arranged in the body (2) in such a way as to take into account a thyroid/neck distance of a human body.

6. The phantom as claimed in one of the preceding claims, in which the cavity (7) is arranged in the body (2) in such a way as to take into account an attenuation of rays emitted by a thyroid in tissues of a human body.

7. The phantom as claimed in one of the preceding claims, in which the fixing means comprise at least one fitting-together system (13, 14) comprising a female portion and a male portion, one of the portions being borne by the first part (3) and the other of the portions being borne by the second part (4) facing the other portion in order to allow the two portions to be fitted together.

8. The phantom as claimed in claim 7, in which the fitting-together system (13, 14) surrounds at least one of the lobes (8, 9) of the cavity.

9. The phantom as claimed in one of the preceding claims, in which the fixing means comprise at least one screw (15) which secures the two body parts (3, 4) together in a sealed manner.

10. The phantom as claimed in claim 9, in which the fixing means further comprise adhesive arranged between the screw and an orifice in the body (2) receiving the screw.

11. The phantom as claimed in one of the preceding claims, in which the filling means comprise a first canal (19) extending from the upper face (16) of the first part (3) as far as into an upper end of a first lobe (8) of the cavity, and a second canal (20) extending from the upper face of the first part (4) as far as into an upper end of a second lobe (9) of the cavity.

12. The phantom as claimed in claim 11, in which the plugging means comprise two screws (21, 22) respectively plugged in one of the canals, and two seals, each seal being arranged in the relevant canal (19, 20) between the associated screw (21, 22) and said canal.

13. The phantom as claimed in one of the preceding claims, in which the phantom comprises a solution filling the cavity of the thyroid phantom.

14. The phantom as claimed in claim 13, in which the solution is a radioactive solution.

15. The phantom as claimed in claim 14, in which the solution comprises at least one radionuclide simulating at least one of the emission lines of iodine 131 and having a longer lifetime than iodine 131.

16. A method for producing a phantom as claimed in one of the preceding claims, comprising the following successive steps:

    - three-dimensional digital modeling of the body of the thyroid phantom (101),
    - separate manufacture of each part of the body by three-dimensional printing using a 3D printer (102),
    - cleaning, off each part of the body thus created, a lost material used by the 3D printer to form the cavity (103),
    - forming the various orifices and housings (105) intended to receive the plugging means and the fixing means,
    - assembling the two parts of the body with one another (106).

17. A thyroid phantom family (200) comprising at least two thyroid phantoms as claimed in one of claims 1 to 15, each thyroid phantom being suited to simulating at least the thyroid of a distinct particular category of human beings.

18. An overall phantom (300) comprising a thyroid phantom as claimed in one of claims 1 to 15, the overall phantom comprising a neck phantom (301) receiving the thyroid phantom, a cervical vertebrae phantom (307) received in the neck phantom and a spinal cord phantom (310) received in the cervical vertebrae phantom.

19. The overall phantom (300) as claimed in claim 18, further comprising a base (312) on which the neck phantom is arranged.

20. An overall phantom family (400) comprising at least two overall phantoms as claimed in claim 18 or claim 19, each overall phantom being suited to simulating at least the thyroid of a distinct particular category of human beings.

Fig. 1

Fig. 2

Fig. 3

Modélisation numérique en trois dimensions du corps — 101

Fabrication des deux parties du corps par impression 3D — 102

Nettoyage des parties du corps du matériau perdu — 103

Formation des orifices et des logements — 104

Nettoyage et dégraissage du corps — 105

Assemblage des deux parties du corps — 106

Remplissage de l'empreinte — 107

Bouchage de l'empreinte — 108

Fig. 4

201    202    203    204

Fig. 5

200

311 ·· A

310

308    309

300    307

306

303    B    304

305

302    301

313    315

16    312

1    12    314

25

Fig. 6

401    402    403    404

201    202    203    204

400

Fig. 7

## RÉFÉRENCES CITÉES DANS LA DESCRIPTION

**Documents brevets cités dans la description**

- JP 2014215222 B

**Littérature non-brevet citée dans la description**

- Absorbed fractions for electron and photon émissions in the developing thyroid : fetus to five years old. *Radiation Protection Dosimetry,* 1998, vol. 79 (1-4), 419-424

- Description of the mathematical phantoms. Oak Ridge National Laboratory, 2005